# EUROPÄISCHE PATENTSCHRIFT

(11) **EP 2 760 499 B1**
(45) Veröffentlichungstag und Bekanntmachung des Hinweises auf die Patenterteilung: **01.11.2017**
(21) Anmeldenummer: 12762015.1
(22) Anmeldetag: 25.09.2012
(51) Int. Cl.: A61M 1/14, A61M 1/16, A61M 1/28, A61M 1/34, A61G 7/00, A61G 7/015, A61G 15/02

(54) **VORRICHTUNG UND SYSTEM ZUR BLUTBEHANDLUNG EINES PATIENTEN**
DEVICE AND SYSTEM FOR TREATING THE BLOOD OF A PATIENT
DISPOSITIF ET SYSTÈME DESTINÉS À TRAITER LE SANG D'UN PATIENT

(30) Priorität: 26.09.2011 DE 102011053935; 26.09.2011 US 201161538991 P
(43) Veröffentlichungstag der Anmeldung: 06.08.2014
(73) Patentinhaber: Fresenius Medical Care Deutschland GmbH, 61352 Bad Homburg (DE)
(72) Erfinder: MEDRANO, Guillermo, 97421 Schweinfurt (DE)
(74) Vertreter: Nordmeyer, Philipp Werner
(86) Internationale Anmeldenummer: PCT/EP2012/068878
(87) Internationale Veröffentlichungsnummer: WO 2013/045448

(56) Entgegenhaltungen:
- DE-A1- 10 141 053
- DE-U1- 29 915 914
- DE-U1-202004 009 683
- DE-U1-202009 016 658

## Beschreibung

### Technisches Gebiet

Die Erfindung betrifft ein Verfahren zur Blutbehandlung eines Patienten, sowie eine Vorrichtung und ein System zur Blutbehandlung.

### Stand der Technik

Verschiedene Verfahren zur Blutbehandlung eines Patienten, insbesondere zur Entwässerung des Blutes eines Patienten, sind bekannt.

Beispielsweise wird bei der Hämodialyse das Blut eines Patienten in einem extrakorporalen Blutkreislauf gereinigt, welcher einen Dialysator umfasst. Der Dialysator weist eine Blutkammer, durch welche das Blut des Patienten geleitet wird, und eine Kammer für Dialysierflüssigkeit auf. Die Blutkammer und die Kammer für die Dialysierflüssigkeit sind durch eine semipermeable Membran voneinander getrennt. Während der Hämodialyse wird die Kammer für die Dialysierflüssigkeit von Dialysierflüssigkeit durchflossen und die Blutkammer von dem Blut des Patienten durchflossen. Aufgrund eines Konzentrationsgradienten zwischen der Dialysierflüssigkeit und dem Blut des Patienten werden entsprechende Substanzen aufgrund der Diffusion durch die semipermeable Membran hindurch transportiert.

Bei der Peritonealdialyse handelt es sich um ein Blutbehandlungsverfahren, welches nicht extrakorporal durchgeführt wird, sondern innerhalb des Patienten. Bei der Peritonealdialyse wird die Bauchhöhle des Patienten über einen durch die Bauchdecke geführten Katheter mit einer Dialyseflüssigkeit befüllt, die einen entsprechenden Konzentrationsgradienten gegenüber den körpereigenen Flüssigkeiten aufweist.

Über das dann als Membran wirkende Bauchfell treten die im Körper vorliegenden Giftstoffe in die in der Bauchhöhle vorliegende Dialyseflüssigkeit über. Nach einiger Zeit, typischerweise einigen Stunden, wird die sich in der Bauchhöhle des Patienten befindliche, nunmehr verbrauchte Dialyseflüssigkeit ausgetauscht. Eine Entwässerung des Patientenblutes findet ebenfalls über das Konzentrationsgefälle der löslichen Stoffe zwischen Blut und Dialyseflüssigkeit statt. Dazu wird die Dialyseflüssigkeit beispielsweise mit Glukose, Dextrinen und/oder anderen Substanzen angereichert. Die Konzentration dieser Stoffe kann über die Zeit hinweg beispielsweise durch Mischvorrichtungen (Pumpen, Ventile, Mischkammern) für Konzentrate einstellbar sein, so dass eine gezielte Entwässerung vorgenommen werden kann. Für eine zumindest teilweise Automatisierung dieses Verfahrens werden in der Praxis spezielle Maschinen verwendet, wie beispielsweise das "Sleep-Safe" Gerät von Fresenius Medical Care.

Eine Blutbehandlung, beispielsweise mit einem der vorstehend aufgeführten Verfahren, ist unter anderem dann notwendig, wenn die Nieren eines Patienten ausfallen. Dann muss eine Dialyse bei dem Patienten so durchgeführt werden, dass Abfallprodukte, so wie beispielsweise Harnstoff, Kreatinin und urämische Toxine, aus dem Blut des Patienten entfernt werden können. Weiterhin werden während der Dialyse überschüssiges Wasser und andere Substanzen, welche üblicherweise harnpflichtig sind, aus dem Körper des Patienten entfernt.

Eine häufig verwendete Form und Verfahren der Dialyse ist die extrakorporale Hämodialyse, bei welcher das Blut des Patienten entlang einer Dialysemembran fließt, wobei auf der anderen Seite dieser semipermeablen Dialysemembran eine Dialysierflüssigkeit bereitgestellt wird, welche in dem Großteil der Verfahren gegenströmig zu dem Blutstrom fließt.

Durch diese semipermeable Membran hindurch werden die Substanzen, welche aus dem Blut des Patienten entfernt werden sollen, aufgrund eines Konzentrationsgradienten zwischen dem Blut und der Dialysierflüssigkeit entfernt, wobei diese Substanzen durch die semipermeable Membran hindurch diffundieren. Dabei können größere Moleküle, deren Diffusionsgeschwindigkeit sehr gering ist, auch konvektiv mittels eines Flüssigkeitsflusses von der Blutseite auf die Seite der Dialyseflüssigkeit durch die semipermeable Membran hindurch transportiert werden.

Die Dialysierflüssigkeit für die extrakorporale Hämodialyse wird dabei typischerweise so bereitgestellt, dass sie eine solche Konzentration an bestimmten aus dem Blut zu entfernenden Substanzen bereitstellt, dass der entsprechende Konzentrationsgradient von der Blutseite zur Seite der Dialyseflüssigkeit hin bereitgestellt wird.

In einem weiteren bekannten Verfahren, nämlich der Hämofiltration, werden bestimmte Substanzen aufgrund eines Druckgradienten über eine Membran eines Dialysefilters abfiltriert. Auch bei diesem Verfahren wird die Porosität bzw. Durchlässigkeit der semipermeablen Membran entsprechend so eingestellt, dass bei dem vorgegebenen Druckgradienten die entsprechenden Substanzen aus dem Blut herausfiltriert werden und über die Membran abgeführt werden. Es ergibt sich jedoch aus den jeweiligen Molekülgrößen, dass bei der Hämofiltration neben den teilweise großen molekularen Substanzen, welche durch die semipermeable Membran hindurch diffundieren müssen, aufgrund des Druckgradienten auch solche Substanzen aus dem Blut heraus gezogen bzw. heraus gefiltert werden, welche physiologisch essentiell für die Blutzusammensetzung sind. Beispielsweise werden dem Blut bei der Hämofiltration Elektrolyte entzogen. Entsprechend werden bei der Hämofiltration die zu viel entnommenen Substanzen sowie das zu viel entnommene Flüssigkeitsvolumen über eine Substitutionsinfusionsflüssigkeit dem Patientenblut nach dem Durchlaufen des Dialysefilters wieder hinzugefügt.

Es gibt auch ein kombiniertes Verfahren, welches als Hämodiafiltration bezeichnet wird, wobei bei diesem Verfahren die oben genannten Stufen der Hämodialyse sowie der Hämofiltration gleichzeitig oder nacheinander in dem extrakorporalen Kreislauf eingesetzt werden.

Bei den Dialyseverfahren wird dem Blut bzw. dem Körper des Patienten neben der Entfernung von harnpflichtigen Substanzen auch überschüssiges Wasservolumen entzogen. Der Wasserentzug aus dem Patientenblut aufgrund einer Druckdifferenz über die Dialysemembran wird als Ultrafiltration bezeichnet. Diese Druckdifferenz, welche zur Erzeugung der Ultrafiltration dient, wird häufig durch eine stromabwärts des jeweiligen Dialysefilters auf der Dialysatseite angeordnete Ultrafiltrationspumpe erzeugt.

Weiterhin ist es bekannt, dass sich ein Patient während der Durchführung der Blutbehandlung bevorzugt in Ruhe befinden sollte, um den Erfolg der Blutbehandlung nicht zu gefährden und um insbesondere das Herz-Kreislaufsystem des Patienten nicht übermäßig zu belasten. Besonders durch einen starken Volumenentzug, d.h. einen starken Entzug von Wasser aus dem Patientenblut, kann eine starke Herz-Kreislaufbelastung auftreten, welche im Extremfall in einer Hypotensionskrise gipfelt. Um dem vorzubeugen wird der Patient bevorzugt in Ruhe gehalten.

Die Lagerung eines Patienten während der Blutbehandlung ist beispielsweise über eine Patientenliege, welche mit einem Blutbehandlungsgerät versehen ist, gemäß der DE 101 41 053 A1 bekannt. Die dort beschriebene Patientenliege ist dazu gedacht, dem Patienten während der Blutbehandlung, welche üblicherweise einige Stunden andauert, eine halbwegs komfortable Sitz-/Liegeposition zu ermöglichen.

### Detaillierte Beschreibung der Erfindung

Entsprechend liegt der vorliegenden Erfindung die Aufgabe zugrunde, eine Vorrichtung und ein System zur Durchführung der Blutbehandlung anzugeben, bei welchem das Auftreten von Herz-Kreislauf-bedingten Komplikationen weiter reduziert wird. Beschrieben wird zunächst ein Verfahren welches jedoch nicht unter den Schutzanspruch fällt. Entsprechend umfasst das Verfahren zur Blutbehandlung eines Patienten eine Entwässerung des Blutes des Patienten bei einer einstellbaren Entwässerungsrate. Erfindungsgemäß wird die Lage des Patienten ermittelt und die Entwässerungsrate wird abhängig von der ermittelten Lage des Patienten eingestellt, gesteuert und/oder geregelt.

Unter Entwässerungsrate wird vorliegend das dem Patientenblut pro Zeiteinheit entzogene Flüssigkeitsvolumen, also insbesondere das dem Patientenblut entzogene Wasservolumen pro Zeiteinheit, verstanden. Je nach angewendetem Blutbehandlungsverfahren (z.B. Hämodialyse, Hämofiltration, Hämodiafiltration, Peritonealdialyse, Apherese und/oder Plasmapherese) ist die Entwässerungsrate der Ultrafiltrationsrate im Wesentlichen dann gleichzusetzen, wenn die Entwässerung des Blutes durch Ultrafiltration erreicht wird.

Es hat sich herausgestellt, dass es bei der Dialyse äußerst wichtig ist, den Flüssigkeitsentzug bzw. die Entwässerungsrate möglichst exakt einzustellen, zu steuern und/oder zu regeln, um die beim Patienten auftretenden Komplikationen bzw. Flüssigkeitsentzugsprobleme, wie beispielsweise Herz-Kreislaufprobleme, möglichst gut kontrollieren bzw. verhindern zu können. Gleichzeitig kann durch die Einstellung, Steuerung und/oder Regelung der Entwässerungsrate dann ein relativ schneller Dialyseprozess erreicht werden, wenn für den jeweiligen Patientenzustand jeweils eine optimale Entwässerungsrate verwendet werden kann.

Durch die Entwässerung kommt es zu einer Abnahme des Plasmavolumens. Falls es der jeweilige menschliche Organismus des Patienten nicht schafft, das Plasmavolumen aus dem interstitiellen Raum wieder aufzufüllen, sinkt entsprechend der Füllungsdruck des Herzens und der Blutdruck fällt ab. Es ist sofort einzusehen, dass dies umso eher geschieht, je höher die jeweilig eingestellte Entwässerungsrate ist. Dabei wird als Blutvolumen die Gesamtmenge des Blutes verstanden, welche sich aus dem Plasmavolumen und dem Erythrozytenvolumen zusammensetzt. Bei einem zu stark absinkenden Blutdruck kommt es zu einer Hypotensionskrise bei dem jeweilig behandelten Patienten.

Es hat sich gezeigt, dass die Körperlage des Patienten das Plasmavolumen beeinflusst. Insbesondere steigt das Plasmavolumen im Liegen an und sinkt demgegenüber im Sitzen bzw. im Stehen ab. Entsprechend kann der Fall auftreten, dass ein Patient, welcher während der Blutbehandlung mit einer konstanten Entwässerungsrate behandelt wird und sich von einer Liegeposition in eine aufrechtere Position bewegt, einen plötzlichen Abfall des Blutdruckes erfährt. Die Körperlage des Patienten beeinflusst also das Plasmavolumen direkt.

Die oben beschriebene Lösung der vorliegenden Aufgabe, nämlich in einem Verfahren zur Blutbehandlung eines Patienten, in welchem das Blut des Patienten mit einer einstellbaren Entwässerungsrate entwässert wird, die Lage des Patienten zu messen und die Entwässerungsrate abhängig von der Lage des Patienten einzustellen, zu regeln und/oder zu steuern, wirkt daher darauf hin, dass, wenn sich der Patient beispielsweise von seiner Liege aufrichtet bzw. aufsetzt, die Entwässerungsrate entsprechend herabgesetzt wird.

Entsprechend kann das Auftreten von Herz-Kreislauf-bedingten Problemen, welche auf die Veränderung der Lage des Patienten zurückzuführen sind, auf diese Weise verringert oder vermieden werden. Mittels des angegebenen Verfahrens zur Blutbehandlung kann daher das Auftreten von kritischen Hypotensionszuständen beim Patienten und insbesondere das Auftreten einer Hypotensionskrise beim Patienten verringert bzw. vermieden werden.

Die Ermittlung der Lage des Patienten wird bevorzugt an einer Patientenliege, an einem Patientenstuhl und/oder an dem Patienten selbst durchgeführt. Bevorzugt wird sie über die Auswertung von an der Patientenliege und/oder dem Patientenstuhl angebrachten Sensoren, von am Patienten angeordneten Sensoren und/oder von den Patienten überwachenden Sensoren, insbesondere der Auswertung von Lagesensoren, optischen Sensoren und/oder bildgebenden Sensoren ermittelt.

Bevorzugt wird die Entwässerung des Blutes mittels einer Peritonealdialyse durchgeführt und die Entwässerungsrate wird durch Einstellen, Steuern und/oder Regeln der Konzentration gelöster Stoffe in der Dialyseflüssigkeit, bevorzugt von Glukose und/oder Dextrin, vor dem Einbringen der Dialyseflüssigkeit in die Bauchhöhle eingestellt, gesteuert und/oder geregelt wird. Um eine weitere Anpassung der Entwässerungsrate an den jeweiligen Zustand des Patienten und insbesondere an den Lagerungszustand des Patienten zu erreichen, wird bevorzugt bei einer Änderung der Lage des Patienten die in der Bauchhöhle vorhandene Dialyseflüssigkeit gegen eine Dialyseflüssigkeit mit einer anderen Konzentration gelöster Stoffe ausgetauscht.

Bevorzugt wird die Entwässerung des Blutes durch Ultrafiltrieren durchgeführt, wobei die Entwässerungsrate durch die Ultrafiltrationsrate (UFR) einstellbar ist und die Ultrafiltrationsrate (UFR) abhängig von der ermittelten Lage des Patienten eingestellt, gesteuert und/oder geregelt wird.

Die Regelung der Entwässerung basierend auf der detektierten Körperlage des Patienten kann zu niedrigeren Dialysezeiten führen, insbesondere wenn der Patient vorwiegend während der Behandlung liegt. Im Liegen stellt sich nämlich in der Regel ein höheres Plasmavolumen beim Patienten ein, weswegen die Entwässerungsrate erhöht werden kann. Eine solchermaßen verkürzte Behandlung schont den Patienten und im Falle der Peritonealdialyse dessen Bauchfell, welches als Filter wirkt.

Die Regelung der Entwässerung basierend auf der detektierten Körperlage des Patienten kann ebenfalls zu niedrigeren Glukosekonzentrationen in der Dialysierflüssigkeit führen, wenn die Körperlage zu einem geringeren Plasmavolumen führt. Eine reduzierte Glukosekonzentration trägt zu einer geringeren Glukosebelastung des Bauchfells bei. Es hat sich gezeigt, dass eine erhöhte Glukosekonzentration in der Dialysierflüssigkeit im Laufe der Zeit bei verschiedenen Patienten zu unerwünschten pathologischen Veränderungen des Bauchfells führen kann, welche im Extremfall die Peritonealdialysebehandlung verhindern können. Eine reduzierte Glukosekonzentration aufgrund der detektierten Körperlage des Patienten kann also dazu beitragen, dass ein Patient länger mit dem Verfahren der Peritonealdialyse behandelt werden kann.

Die Einstellung, Steuerung und/oder Regelung der Ultrafiltrationsrate kann mit Vorteil durch Einstellung, Steuerung und/oder Regelung der Pumprate bei der Ultrafiltration erreicht werden, insbesondere durch Einstellung, Steuerung und/oder Regelung der Pumprate einer stromabwärts eines Dialysefilters angeordneten Schlauchrollenpumpe, Zahnradpumpe, Membranpumpe und/oder Impellerpumpe.

In einer bevorzugten Variante wird die Entwässerungsrate, bevorzugt die Ultrafiltrationsrate, bei einem im Wesentlichen liegenden Patienten höher eingestellt, gesteuert und/oder geregelt, als bei einem gegenüber der liegenden Position aufrechter angeordneten Patienten. Auf diese Weise kann die Entwässerungsrate genau an die physiologischen Bedingungen angepasst werden, so dass eine komplikationsarme und effiziente Entwässerung des Patientenblutes erreicht werden kann.

Bevorzugt wird die Entwässerungsrate gegenüber der vorhergehenden Entwässerungsrate erhöht, wenn sich die Lage des Patienten mehr einer liegenden Haltung annähert, und die Entwässerungsrate wird verringert, wenn sich die Lage des Patienten gegenüber der vorhergehenden Lage mehr in Richtung einer aufrechten Lage verändert.

Hierzu kann bevorzugt eine Lagemessung an einer Patientenliege, einem Patientenstuhl bzw., über entsprechende Sensoren und/oder bildgebende Mittel, am Patienten selbst ermittelt werden.

In einer weiteren bevorzugten Ausführungsvariante kann der Patient, insbesondere über Aktoren an einer entsprechenden von ihm benutzten Patientenliege bzw. Patientenstuhl, seine Körperlage selbst einstellen. Hierdurch wird erreicht, dass die relativ lange Behandlungszeit bei der Blutbehandlung für den Patienten so bequem wie möglich gestaltet werden kann. Insbesondere kann der Patient immer wieder selbständig seine Körperlage anpassen. Bei der vom Patienten selbst gesteuerten Anpassung der Körperlage wird dann gleichzeitig die Entwässerungsrate, bevorzugt die Ultrafiltrationsrate, so angepasst, dass es auch bei einer veränderten Körperlage nicht zu Problemen, beispielsweise einer Hypotensionskrise des Patienten, kommt. Durch die weitgehende Selbstbestimmung des Patienten seiner eigenen Lage kann entsprechend die Behandlung für den jeweiligen Patienten angenehmer gestaltet werden.

Das Verfahren wird bevorzugt bei der Hämodialyse, der Hämofiltration, der Hämodiafiltration, der Peritonealdialyse, der Apherese sowie der Plasmapherese verwendet.

Um eine zügige und komplikationsarme Blutbehandlung des Patienten zu erreichen, wird die Entwässerungsrate, bevorzugt die Ultrafiltrationsrate, zu Beginn der Behandlung bevorzugt höher eingestellt, gesteuert und/oder geregelt, als in einem nachfolgenden Abschnitt der Behandlung. Dadurch kann auch den physiologischen Gegebenheiten dahingehend Rechnung getragen werden, dass das überschüssige Plasmavolumen zu Beginn der Behandlung am größten ist.

In einer weiteren vorteilhaften Ausgestaltung des Verfahrens wird die Entwässerungsrate, bevorzugt die Ultrafiltrationsrate, nur in einem ersten Behandlungsabschnitt abhängig von der Lage des Patienten gesteuert und/oder geregelt, wird aber in einem zweiten Behandlungsabschnitt konstant gehalten, wobei der erste Behandlungsabschnitt bevorzugt durch das Entziehen der Hälfte der geplanten Flüssigkeitsmenge endet. Entsprechend gleicht der zweite Abschnitt der Behandlung im Wesentlichen der herkömmlichen Behandlung.

In einer weiteren vorteilhaften Ausbildung des Verfahrens wird die Entwässerungsrate, bevorzugt die Ultrafiltrationsrate, zur Einstellung, Steuerung und/oder Regelung abhängig von der Lage des Patienten um eine vorgegebene Differenz (UFR+) von einer vorgegebenen Entwässerungsrate, bevorzugt Ultrafiltrationsrate (UFR_{SET}), angehoben und/oder abgesenkt, wobei die vorgegebene Differenz (UFR+) bevorzugt aus der zu erwartenden Plasmavolumenänderung zwischen im Wesentlichen liegender und im Wesentlichen aufrechter Position des Patienten abgeleitet wird. Auf diese Weise kann das Auftreten einer lagebedingten Hypotensionskrise besonders gut vermieden werden, da die Entwässerungsrate an die zu erwartende Plasmavolumenänderung angepasst wird. Das angegebene Verfahren führt entsprechend dazu, dass die Entwässerungsrate genau an die erwartete Plasmavolumenänderung angepasst wird und entsprechend eine sichere und effiziente Entwässerung durchgeführt werden kann.

In einer weiteren Ausgestaltung des Verfahrens wird wobei die Entwässerungsrate, bevorzugt die Ultrafiltrationsrate (UFR), zunächst für einen Zeitabschnitt (t_{UFR±}) der Behandlung angehoben, bevorzugt um einen vorgegebenen Wert (UFR+), und dann bei Auftreten einer Lageänderung des Patienten in Richtung einer aufrechteren Lage, für den gleichen Zeitabschnitt (t_{UFR±}) abgesenkt, bevorzugt um den gleichen vorgegebenen Wert (UFR+), wobei bevorzugt zunächst eine erhöhte Entwässerungsrate, bevorzugt Ultrafiltrationsrate (UFR=UFR_{SET} + UFR+), und dann eine erniedrigte Entwässerungsrate, bevorzugt Ultrafiltrationsrate (UFR=UFR_{SET} - UFR+), eingestellt, geregelt und/oder gesteuert wird. Durch die Verwendung der zunächst erhöhten und dann abgesenkten Entwässerungsrate kann eine Behandlung bereitgestellt werden, bei der die Behandlungsdauer gegenüber den konventionellen Verfahren nicht verlängert wird, aber die Sicherheit bezüglich eventueller Hypotensionskrisen erhöht wird.

In einer weiteren vorteilhaften Ausprägung wird die Entwässerungsrate am Anfang der jeweiligen Behandlung gegenüber dem üblicherweise verwendeten Wert erhöht und erst im Laufe der Behandlung entsprechend an die Lage des Patienten angepasst. Insbesondere kann die Entwässerungsrate erst nach dem Überschreiten eines bestimmten Aufrichtungswinkels des Patienten, beispielsweise einem Winkel des Patientenoberkörpers von größer als 30° gegenüber der Horizontalen angepasst werden. In einem Bereich zwischen einer komplett horizontalen Ausrichtung des Körpers des Patienten und diesem Winkel wird entsprechend keine Anpassung vorgenommen.

Weiterhin kann die jeweilige Entwässerungsrate auch unter Berücksichtigung des typischen Patientenverhaltens gewählt werden, also unter Berücksichtigung der Zeit, welche ein Patient üblicherweise in einer eher aufgerichteten Lage verbringt. Entsprechend kann, um die Gesamtbehandlungszeit einzuhalten, die Entwässerungsrate zu Beginn der Behandlung sowie in den Abschnitten, in welchen der Patient sich in einer eher aufrechten Lage befindet, um einen Betrag erhöht werden, welcher sich aus der Vorhersage der voraussichtlich im eher aufgerichteten Zustand verbrachten Zeit ableitet.

Die oben beschriebene Aufgabe wird weiterhin durch eine Vorrichtung mit den Merkmalen des Anspruchs 1 gelöst. Vorteilhafte Ausgestaltungen finden sich in den Unteransprüchen.

Entsprechend umfasst die Vorrichtung zur Blutbehandlung eines Patienten eine Blutbehandlungsvorrichtung zum Entwässern des Blutes des Patienten bei einer einstellbaren Entwässerungsrate. Erfindungsgemäß sind Lageermittelungsmittel zur Ermittlung der Lage des Patienten vorgesehen, wobei die Entwässerungsrate abhängig von der ermittelten Lage des Patienten einstellbar, regelbar und/oder steuerbar ist.

Bevorzugt sind die Lageermittelungsmittel als an der Patientenliege und/oder dem Patientenstuhl angebrachte Sensoren, als am Patienten angeordnete Sensoren und/oder als den Patienten überwachende Sensoren ausgebildet und sind bevorzugt als Lagesensoren, optische Sensoren und/oder bildgebende Sensoren ausgebildet.

Um dem Patienten einen hohen Behandlungskomfort zu ermöglichen, sind auf eine Patientenliege und/oder einen Patientenstuhl wirkende Aktoren vorgesehen, über welche der Patient seine Lage selbst einstellen kann.

Eine Datenverarbeitungsvorrichtung zur Auswertung der Signale der Lageermittelungsmittel kann vorgesehen sein, wobei die Datenverarbeitungsvorrichtung die Entwässerungsrate, bevorzugt die Ultrafiltrationsrate (UFR), entsprechend der ermittelten Lage des Patienten regeln und/oder steuern kann.

Die Blutbehandlungsvorrichtung ist bevorzugt zur Durchführung der Hämodialyse, der Hämofiltration, der Hämodiafiltration, der Peritonealdialyse, der Apherese und/oder der Plasmapherese ausgebildet.

Weiterhin wird die oben beschriebene Aufgabe durch ein System mit den Merkmalen des Anspruchs 15 gelöst.

### Kurze Beschreibung der Figuren

Im Folgenden werden bevorzugte Ausführungsformen der vorliegenden Erfindung anhand der nachfolgenden Figuren beschrieben:
- Figur 1: zeigt schematisch eine Vorrichtung zur Blutbehandlung mit einer Patientenliege und einer Blutbehandlungsvorrichtung;
- Figur 2: zeigt den Verlauf der Ultrafiltration in einem herkömmlichen Verfahren gemäß dem Stand der Technik;
- Figur 3: zeigt schematisch den Verlauf der Ultrafiltration in einem Verfahren gemäß einer Ausprägung der vorliegenden Erfindung; und
- Figur 4: zeigt schematisch einen exemplarischen Algorithmus zur Durchführung des vorliegenden Verfahrens.

### Ausführliche Beschreibung der Figuren

Die vorliegende Erfindung wird im Folgenden anhand der Figuren detailliert beschrieben werden. Dabei werden gleiche Bezugszeichen für ähnliche oder gleiche Bestandteile verwendet und auf eine wiederholte Beschreibung in den unterschiedlichen Ausführungsbeispielen kann verzichtet werden, um Redundanzen zu vermeiden.

Nachfolgend wird das Verfahren, die Vorrichtung sowie das System anhand des Beispiels der Hämodialyse exemplarisch beschrieben. Diese Beschreibung ist jedoch nicht beschränkend zu verstehen, sondern das Verfahren sowie die Vorrichtung und das System können in jeder anderen Blutbehandlung verwendet werden, insbesondere der Hämodialyse, der Hämofiltration, der Hämodiafiltration, der Peritonealdialyse, der Apherese und/oder der Plasmapherese.

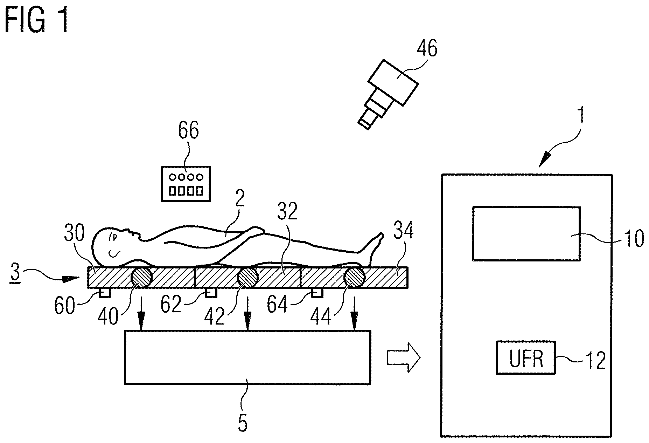

In Figur 1 ist schematisch eine Vorrichtung zur Blutbehandlung eines Patienten nach einer bevorzugten Ausführungsform der vorliegenden Erfindung gezeigt.

Hierzu ist eine Blutbehandlungsvorrichtung 1 in Form einer Hämodialysemaschine vorgesehen, mittels welcher der Patient 2 einer Blutwäsche unterzogen werden kann. Die Hämodialysemaschine umfasst eine Entwässerungsvorrichtung in Form des Ultrafiltrationsabschnitts 10, welcher über eine Einstellvorrichtung 12 mit einer einstellbaren Entwässerungsrate in Form einer einstellbaren Ultrafiltrationsrate UFR betrieben werden kann.

Entsprechend kann über die Einstellung der Ultrafiltrationsrate UFR an der Blutbehandlungsvorrichtung 1 der Flüssigkeitsentzug beim Patienten 2 während der Behandlung eingestellt werden. Die Ultrafiltrationsrate UFR ergibt sich dabei als Volumen der über die Ultrafiltration entzogenen Flüssigkeit, welches hier als UFV bezeichnet wird, pro Zeit t, also als UFR=UFV/t. Allgemein ergibt sich die Entwässerungsrate als das dem Patientenblut pro Zeiteinheit entzogene Wasservolumen.

Der Patient 2 ist auf einer Patientenliege 3 gelagert. Die Patientenliege 3 umfasst in der gezeigten Ausführungsform ein Kopfteil 30, ein Mittelteil 32 und ein Fußteil 34. Das Kopfteil 30, das Mittelteil 32 sowie das Fußteil 34 können relativ zueinander in unterschiedliche Positionen gebracht werden, so dass der Patient 2 von der in Figur 1 gezeigten vollständig horizontal ausgerichteten - also liegenden - Position weiter aufgerichtet werden kann. Der Patient 2 kann beispielsweise durch Anheben bzw. Schrägstellen des Kopfteiles 30 so aufgerichtet werden, dass sein Oberkörper in eine gegenüber der liegenden Position aufrechtere Position gebracht wird. Das Fußteil 34 kann beispielsweise gleichzeitig abgesenkt werden, so dass die einzelnen Komponenten der Patientenliege 3 dann eine Lagerungsposition für den Patienten 2 ergeben, in welcher der Patient 2 eher eine sitzende Position einnimmt, also mit angehobenem Oberkörper und abgesenkten Beinen. Das Fußteil 34 kann aber auch angehoben werden, um bei Herz-Kreislaufproblemen entsprechend das Blutvolumen aus den Beinen dem Kernbereich des Patienten 2 wieder zuzuführen.

Die Patientenliege 3 kann weiterhin als Ganzes leicht gekippt werden, also der Patient 2 insgesamt in eine schräge Position gebracht werden, um dem Patienten 2 so ein schräggestelltes Bett bereit zu stellen.

Bei der in der Figur 1 gezeigten Patientenliege 3 handelt es sich im Prinzip um eine herkömmliche Patientenliege, welche die entsprechenden Einstellmöglichkeiten bereitstellt. Die einzelnen Teile der Patientenliege 3 können dabei über Aktoren 60, 62, 64 relativ zueinander verstellt werden, um eine einfache Änderung der Lagerung des Patienten 2 ermöglichen. Die Aktoren 60, 62, 64 sind typischer Weise in Form von Elektromotoren, pneumatischen Antrieben und/oder hydraulischen Antrieben gegeben. In einer bevorzugten Variante kann der Patient 2 die Aktoren 60, 62, 64 über ein entsprechendes Schaltpaneel 66 selbstständig betätigen, um eine für ihn bequeme Position einstellen zu können.

Bevorzugt ist an der Patientenliege 3, zumindest zur Verwendung der Patientenliege 3 in einer aufgerichteten bzw. einer der Sitzposition ähnlichen Positionierung, auch eine Armaufnahme, die in Figur 1 nicht gezeigt ist, vorgesehen, um dem Patienten 2 Armstützen zu bieten.

Die Patientenliege 3 ist in Figur 1 lediglich als aus drei Abschnitten, nämlich aus dem Kopfteil 30, dem Mittelteil 32 und dem Fußteil 34 bestehend, gezeigt. Die Patientenliege 3 kann jedoch beliebig komplex ausgebildet sein und insbesondere noch mehr gegeneinander verstellbare Abschnitte als die in Figur 1 gezeigten aufweisen, beispielsweise neben einem separaten Kopfteil auch ein verstellbares Rückenteil oder eine geteilte Fußablage, um weitere unterschiedliche Positionen des Patienten 2 zu ermöglichen, um diesem eine möglichst bequeme Lagerung während der Blutbehandlung zu ermöglichen. Hierbei ist festzuhalten, dass die Blutbehandlung typischer Weise relativ lange dauert - Behandlungsdauern von 3 bis 7 Stunden sind üblich. Daher ist es für das Wohlergehen des Patienten 2 besonders wichtig, dass er sich für diese Zeit eine bequeme Position verschaffen kann.

Auch bei der in Figur 1 gezeigten Blutbehandlungsvorrichtung 1 in Form einer Hämodialysemaschine handelt es sich um eine weitgehend herkömmliche Hämodilysemaschine, bei welcher die Ultrafiltrationsrate UFR über eine entsprechende Einstelleinrichtung 12 eingestellt werden kann.

Die Blutbehandlungsvorrichtung 1 kann auch zur Durchführung der Hämofiltration, der Hämodiafiltration, der Peritonealdialyse, der Apherese und/oder der Plasmapherese ausgebildet sein.

An der Patientenliege 3 sind Sensoren 40, 42, 44 vorgesehen, mittels welchen die Lage der einzelnen Teile der Behandlungsliege 3 bezüglich der Horizontalen und/oder relativ zueinander bestimmt werden kann. Weiterhin ist eine Kamera 46 vorgesehen, welche ebenfalls zur Ermittlung der aktuellen Lage des Patienten 2 verwendet wird.

Mittels der Auswertung der Signale der Sensoren 40, 42, 44, 46 kann entsprechend in einer Datenauswertungsvorrichtung 5 die genaue Lage des Patienten 2 bezüglich der Horizontalen ermittelt werden. Die Ableitung der Lage bzw. Position des Patienten 2 aus der jeweiligen Einstellung der Patientenliege 3 setzt natürlich voraus, dass sich der Patient 2 in der durch die Form der einzelnen Teile der Patientenliege 3 vorgegebenen Lage befindet. Mit anderen Worten wird davon ausgegangen, dass der Patient 2 im Wesentlichen an den einzelnen Komponenten der Patientenliege 3 in der vorgesehenen Weise anliegt bzw. auf diesen Komponenten aufliegt.

Wenn sich der Patient 2 hingegen beispielsweise bei der in Figur 1 gezeigten Positionierung der Patientenliege 3 selbstständig aufrichten sollte, und entsprechend der Oberkörper keinen Kontakt mehr zur Patientenliege 3 hat, kann selbstverständlich aus der Position der Patientenliege 3 nicht auf die Position des Patienten 2 geschlossen werden. Nachfolgend wird jedoch angenommen, dass sich der Patient während der Blutbehandlung stets in Kontakt mit den jeweiligen Komponenten der Patientenliege 3 befindet.

In einer Variante oder Ergänzung kann die Lage des Patienten 2 auch über am Patienten direkt angebrachte Sensoren ermittelt werden, beispielsweise mittels in die jeweiligen, vom Patienten 2 getragenen Textilien eingearbeitete Sensoren.

In einer anderen bevorzugten Variante oder Ergänzung können optische Sensoren und/oder bildgebende Sensoren wie beispielsweise die gezeigte Kamera 46 dazu verwendet werden, die Lage des Patienten 2 zu ermitteln. Hierfür bieten sich beispielsweise eine Lichtschrankenmessung oder Laserscannermessung an, oder aber eine Bildaufzeichnung bzw. Videoaufzeichnung des Patienten während der Blutbehandlung, aus welcher die jeweilige Lage des Patienten 2 ermittelt werden kann.

In der Datenverarbeitungsvorrichtung 5 wird entsprechend über die in Figur 1 gezeigten Sensoren 40, 42, 44, 46, bzw. über die in Figur 1 nicht gezeigten, möglichen weiteren Sensoren, die Lage des Körpers des jeweiligen Patienten 2 ermittelt.

Abhängig von der in der Datenverarbeitungsvorrichtung 5 ermittelten Lage des Patienten 2 wird die Entwässerungsrate in der Dialysemaschine 1 eingestellt. Entsprechend wird bei der hier exemplarisch gezeigten Hämodialysevorrichtung 1 mit dem Ultrafiltrationsabschnitt 10 die Ultrafiltrationsrate UFR entsprechend eingestellt.

Da sich gezeigt hat, dass sich das Plasmavolumen eines Patienten 2 bei einer Lageveränderung ändert, kann auf diese Weise erreicht werden, dass die Ultrafiltrationsrate UFR stets optimal an den jeweiligen Zustand des Patienten 2 angepasst ist. Auf diese Weise können nachteilige Effekte auf die Stabilität des Kreislaufes des behandelten Patienten verringert bzw. vermieden werden.

Insbesondere wurde herausgefunden, dass das Plasmavolumen eines Patienten 2 ansteigt, wenn er von einer aufrechteren Lage in eine liegendere Lage gebracht wird, und das Plasmavolumen absinkt, wenn der Patient 2 von einer liegenderen Lage in eine aufrechtere Lage gebracht wird.

Die Entwässerungsrate, insbesondere die Ultrafiltrationsrate UFR, wird entsprechend von der Datenverarbeitungsvorrichtung 5 aufgrund der ermittelten Lage bzw. der jeweiligen Lageveränderung des Patienten 2 eingestellt, geregelt und/oder gesteuert.

Die jeweilig durchgeführte Veränderung der Entwässerungsrate bzw. der Ultrafiltrationsrate UFR in Reaktion auf eine Veränderung der Lage des Patienten 2 kann dabei beispielsweise über die erwartete Veränderung des Plasmavolumens zwischen einer liegenden und einer aufrechten Position des Patienten 2 berechnet werden. Als Alternative wird eine Relation aus der Lageveränderung des Patienten 2 und einer statistisch ermittelten Anpassung der Entwässerungsrate verwendet. Diese statistische Auswertung kann entweder für mehrere Patienten, oder aber für den jeweiligen individuellen Patienten durchgeführt werden. Zur Bestimmung dieser Daten können beispielsweise in den ersten Sitzungen eines Patienten auch andere Parameter überwacht werden, beispielsweise der Blutdruck des Patienten. Aus der Korrelation der jeweiligen Parameter, insbesondere der Lage des Patienten und dem Blutdruck, kann dann der entsprechende Anpassungsfaktor berechnet werden.

Die von der Datenverarbeitungsvorrichtung 5 durchgeführte Einstellung, Steuerung bzw. Regelung der Entwässerungsrate aufgrund der ermittelten Lage des Patienten 2 wird entsprechend entweder über eine feste mathematische Relation zwischen der Lage des Patienten 2 und der Entwässerungsrate erreicht, oder aber durch Ablesen aus einer statistisch erzeugten Tabelle für bestimmte Lagebereiche durchgeführt.

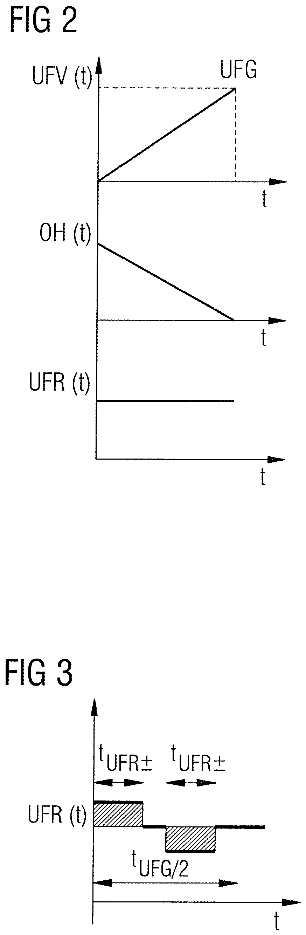

Figur 2 zeigt schematisch den Ablauf einer herkömmlichen Dialysebehandlung mittels einer Hämodialysevorrichtung mit einem Ultrafiltrationsabschnitt nach dem Stand der Technik, wobei im obersten Diagramm das gesamte Ultrafiltrationsvolumen über die Zeit aufgetragen ist als UFV(t). Hierbei ist zu erkennen, dass das Ultrafiltrationsvolumen, also das dem Patienten entnommene Flüssigkeitsvolumen, über die Zeit hinweg quasi linear ansteigt bis das gewünschte bzw. geplante Ultrafiltrationsvolumen UFG erreicht ist.

In dem darunter liegenden mittleren Diagramm ist das überschüssige Wasservolumen im Patienten 2 über die Zeit hinweg als OH(t) aufgetragen. Am Ende der jeweiligen Blutbehandlung sollte das überschüssige Wasservolumen OH im Patienten auf Null abgesunken sein, dann ist, wie aus dem darüber liegenden Diagramm abzuleiten, das gewünschte Ultrafiltrationsvolumen UFG erreicht.

Im untersten Diagramm ist die Ultrafiltrationsrate über die Zeit angegeben als UFR(t). Es ist direkt zu erkennen, dass die Ultrafiltrationsrate in dem herkömmlichen Verfahren konstant gehalten wird.

Figur 3 zeigt nun einen möglichen Verlauf der Entwässerungsrate, insbesondere der Ultrafiltrationsrate, über die Zeit als UFR(t) gemäß einem möglichen Ausführungsbeispiel der vorliegenden Erfindung. Es ist zu erkennen, dass die Ultrafiltrationsrate UFR(t) in diesem gezeigten Ausführungsbeispiel variabel ist. Insbesondere wird die Ultrafiltrationsrate UFR(t) zu Beginn der Behandlung über einen Zeitabschnitt t_{UFR±} hinweg zunächst um einen Betrag UFR+ erhöht.

Eine wesentliche Beeinträchtigung des Herz-Kreislaufsystems des Patienten 2 wird in dieser allerersten Phase der Blutbehandlung nicht erwartet, da hier das Plasmavolumen noch am höchsten ist und entsprechend eine Verringerung des Plasmavolumens mit einer erhöhten Entwässerungsrate bzw. Ultrafiltrationsrate UFR nicht zu einer Hypotoniekrise führt. Darüber hinaus befindet sich der Patient 2 zu Beginn der Behandlung üblicher Weise in einer Liegeposition.

Die Zeit t_{UFR±} ist eine statisch ermittelte Zeit, über welche hinweg ein Patient typischerweise eine aufrechtere Stellung einnimmt, also beispielsweise eine Neigung des Oberkörpers von größer als 30° gegenüber der Horizontalen einnimmt. Der Wert t_{UFR±} wird entweder aus einer Statistik einer bestimmten Anzahl unterschiedlicher Patienten bestimmt, oder kann aus den vorvergangenen Sitzungen des jeweiligen individuellen Patienten 2 bestimmt werden. Hierbei ist zu berücksichtigen, dass Dialysepatienten üblicherweise zwei bis drei Mal in der Woche eine Dialysesitzung wahrnehmen müssen und entsprechend schnell eine signifikante Datenlage für jeden individuellen Patienten zusammenkommt.

Die Entwässerungsrate bzw. Ultrafiltrationsrate UFR wird dann, wie sich aus Figur 3 ergibt, später dann um den Wert UFR+ abgesenkt, wenn der Patient eine Lageveränderung vornimmt. Die Absenkung der Entwässerungsrate bzw. Ultrafiltrationsrate UFR wird dabei wieder über den vorher bestimmten Zeitraum t_{UFR±} hinweg vorgenommen, nachdem der Patient die Lageänderung vorgenommen hat.

In dem hier gezeigten Ausführungsbeispiel kann in Figur 3 eine Anpassung an die jeweilige Lageveränderung nur solange vorgenommen, wie die Hälfte des geplanten Entwässerungsvolumens bzw. Ultrafiltrationsvolumens UFG noch nicht erreicht ist. Mit anderen Worten findet eine Anpassung nur dann statt, wenn die der gewünschten halben Ultrafiltrationsgesamtmenge UFG/2 entsprechende Zeit, welche hier als t_{UFG/2} bezeichnet wird, noch nicht erreicht ist. Die Zeit, bei welcher die Hälfte des Entwässerungsvolumens bzw. Ultrafiltrationsvolumens erreicht ist, wird als t_{UFG/2} bezeichnet.

Nach Ablauf dieser Zeit wird dann die ursprünglich festgelegte Entwässerungsrate bzw. Ultrafiltrationsrate, welche nachfolgend als UFR_{SET} bezeichnet wird, für die nachfolgende Entwässerung verwendet.

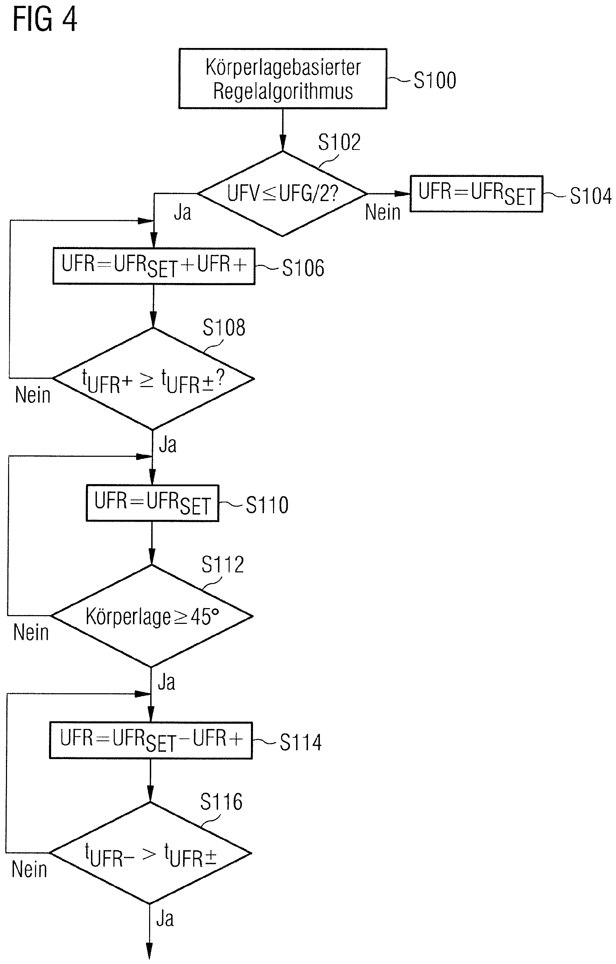

Figur 4 zeigt einen entsprechenden Algorithmus, mittels welchem die Entwässerungsrate festgelegt wird.

Zunächst wird als Schritt S100 der körperlagenbasierte Regelalgorithmus angegeben.

In einer ersten Abfrage wird im Schritt S102 festgestellt, ob das bereits erreichte Entwässerungsvolumen bzw. Ultrafiltrationsvolumen UFV noch kleiner als die Hälfte des geplanten Entwässerungsvolumens bzw. Ultrafiltrationsvolumens UFG ist, also die Abfrage UFV≤UFG/2. Wenn diese Bedingung nicht erfüllt ist, also das bereits erreichte Entwässerungsvolumen bzw. Ultrafiltrationsvolumen UFV des Patienten größer ist als die Hälfte des geplanten Entwässerungsvolumens bzw. Ultrafiltrationsvolumens UFG, wird im Schritt S104 die Entwässerungsrate bzw. Ultrafiltrationsrate auf die ursprünglich festgelegte Entwässerungsrate bzw. Ultrafiltrationsrate gesetzt, also UFR=UFR_{SET}.

Wenn das bereits erreichte Entwässerungsvolumen bzw. Ultrafiltrationsvolumen UFV noch kleiner als die Hälfte des geplanten Entwässerungsvolumens bzw. Ultrafiltrationsvolumens UFG ist, wird die Entwässerungsrate bzw. Ultrafiltrationsrate im Schritt S106 auf einen erhöhten Wert, nämlich um einen an die Veränderung der Körperlage angepassten Veränderungswert der Entwässerungsrate bzw. Ultrafiltrationsrate, welche hier als UFR+ bezeichnet wird, hochgesetzt. Entsprechend beträgt die Entwässerungsrate bzw. Ultrafiltrationsrate im Schritt S106 dann UFR=UFR_{SET} + UFR+.

In der nachfolgenden Abfrage im Schritt S108 wird überprüft, ob die Zeit, in welcher diese Erhöhung der Entwässerungsrate bzw. Ultrafiltrationsrate durchgeführt wird, noch kleiner ist, als die erwartete Zeit der aufrechten Lage des Patienten. Entsprechend wird überprüft, ob t_{UFR+} ≥ t_{UFR±}. Ist diese Zeit noch nicht abgelaufen, so wird die Erhöhung der Entwässerungsrate bzw. Ultrafiltrationsrate um den Wert UFR+ beibehalten. Wenn diese Zeit abgelaufen ist, wird die Entwässerungsrate bzw. Ultrafiltrationsrate auf die vorbestimmte Entwässerungsrate bzw. Ultrafiltrationsrate UFR_{SET} erniedrigt. Entsprechend wird im Schritt S110 UFR=UFR_{SET} gesetzt.

In der nächsten Abfrage im Schritt S112 wird überprüft, ob sich die Körperlage des Patienten in der Zwischenzeit geändert hat, insbesondere auf einen Wert von ≥ 45°, also der Patient sich aufgerichtet hat.

Wenn dies der Fall ist, wird im Schritt S114 die Entwässerungsrate bzw. Ultrafiltrationsrate herabgesetzt, nämlich wiederum um den Betrag UFR+, so dass im Schritt S114 die Entwässerungsrate bzw. Ultrafiltrationsrate gesetzt wird auf UFR=UFR_{SET} - UFR+.

In der nächsten Abfrage beim Schritt S116 wird dann überprüft, ob die Zeit für die Herabsetzung der Entwässerungsrate bzw. Ultrafiltrationsrate, nämlich T_{UFR-}, bereits der vorausbestimmten Zeit für die durchschnittliche Verweildauer des Patienten in der aufgerichteten Lage, nämlich t_{UFR±}, bereits abgelaufen ist. Wenn dies nicht der Fall ist, also T_{UFR-} ≥ t_{UFR±}, wird die Entwässerungsrate bzw. Ultrafiltrationsrate auf dem um den Betrag UFR+ abgesenkten Wert gehalten. Sobald jedoch diese Zeit abgelaufen ist, wird die Entwässerungsrate bzw. Ultrafiltrationsrate wieder auf der festgelegten Entwässerungsrate bzw. Ultrafiltrationsrate gehalten, also auf UFR_{SET}.

Vorteilhaft an diesem Algorithmus ist, dass ein Zeitverlust bzw. eine Verlängerung der Behandlungsdauer nicht auftritt, da der Abschnitt der reduzierten Entwässerungsrate bzw. Ultrafiltrationsrate (UFR=UFR_{SET} - UFR+) kompensiert wird durch den anfänglichen Abschnitt mit der erhöhten Entwässerungsrate bzw. Ultrafiltrationsrate (UFR=UFR_{SET} + UFR+). Dabei wird jedoch angenommen, dass der Patient zu Beginn der Behandlung liegt und entsprechend ein hohes Plasmavolumen vorhanden ist, so dass Kreislaufprobleme auch bei der erhöhten Entwässerungsrate bzw. Ultrafiltrationsrate nicht auftreten. Zu einem späteren Zeitpunkt, vor der Hälfte der Behandlungsdauer, kann jedoch die Behandlung bezüglich des Auftretens einer Hypotoniekrise dadurch unterstützt und verbessert werden, dass die Entwässerungsrate bzw. Ultrafiltrationsrate bei Aufsetzen des Patienten verringert wird.

Der in Figur 4 gezeigte Algorithmus funktioniert besonders gut, wenn die Lage des Patienten nur einmal während der Behandlung von einer liegenden Position in eine aufrechte Position verändert wird. Die Weiterbildung dieses Algorithmus für eine ständige Überwachung und eine entsprechende Anpassung der Entwässerungsrate an die jeweilige Lage des Patienten ist für den Fachmann jedoch sofort durchführbar. Insbesondere kann, besonders dann, wenn die Verlängerung der Behandlung unproblematisch ist, eine direkte Korrelation zwischen dem Lagewinkel des Patienten zur Horizontalen und der Entwässerungsrate hergestellt werden. Hierbei ist bei liegenden Patienten, also einem Winkel von 0°, eine höhere Entwässerungsrate möglich, als bei einem aufgerichteten Patienten, also beispielsweise bei einer Position der Rückenlehne von größer als 30° und nahe 90°. Die entsprechende Relation kann hier durch eine statistische Auswertung über unterschiedliche Patienten hinweg gefunden werden, oder aus historisch-statistischen Daten des jeweiligen individuellen Patienten.

In einer besonders bevorzugten Variante kann der Patient 2 über das Schaltpaneel 66 selbst bestimmen, in welcher Lage er die jeweiligen Zeitabschnitte seiner Blutbehandlung durchführen möchte. Der Patient kann hier beispielsweise über das Schaltpaneel 66 die jeweiligen Positionen der Patientenliege 3 in bekannter Art und Weise einstellen, so dass er jeweils die für ihn bequemste Position einnehmen kann.

Die vom Patienten vorgewählte bzw. eingestellte Lage der Patientenliege wird dann von der Datenverarbeitungsvorrichtung 5 dazu verwendet, die Entwässerungsrate an die entsprechende Lage anzupassen. Auch hier wird wiederum je nach Winkel des Patienten bezüglich der Horizontalen eine unterschiedliche Entwässerungsrate eingestellt, wobei die Entwässerungsrate für einen liegenden Patienten höher ist als die Entwässerungsrate bei einem aufrecht sitzenden Patienten. Auf diese Weise kann die Behandlung für den Patienten 2 deutlich angenehmer gestaltet werden, da er über seine Lage frei verfügen kann.

Die Behandlungsdauer kann durch die freie Wahl der Lage unwesentlich verlängert werden. Die Entwässerungsrate kann jedoch bei liegendem Patienten gegenüber der sonst üblichen Entwässerungsrate erhöht werden, da die sonst üblicherweise fest eingestellte Entwässerungsrate sowohl für den liegenden Patienten als auch für den sich in einer aufrechteren Position befindlichen Patienten vorgegeben ist. Ein sich in liegender Position befindlicher Patient kann jedoch eine höhere Entwässerungsrate verkraften, ohne dass es hier zu einer Hypotoniekrise kommt. Entsprechend kann auch bei einer solchen Einstellung und bei einem Vorwählen der jeweiligen Lage durch den Patienten in direkter Ankopplung an die Entwässerungsrate eine Behandlungsdauer erreicht werden, die für den Patienten erträglich und für die jeweilige medizinische Einrichtung wirtschaftlich tragbar ist.

## Patentansprüche

1. Vorrichtung zur Blutbehandlung eines Patienten (2), umfassend eine Blutbehandlungsvorrichtung (1) zum Entwässern des Blutes des Patienten (2) bei einer einstellbaren Entwässerungsrate,
**gekennzeichnet durch**
Lageermittelungsmittel (40, 42, 44, 46) zur Ermittlung der Lage des Patienten (2), wobei die Entwässerungsrate abhängig von der ermittelten Lage des Patienten einstellbar, regelbar und/oder steuerbar ist.

2. Vorrichtung gemäß Anspruch 1, wobei die Lageermittelungsmittel als an einer Patientenliege (3) und/oder einem Patientenstuhl angebrachte Sensoren (40, 42, 44), als am Patienten (2) angeordnete Sensoren und/oder als den Patienten überwachende Sensoren ausgebildet sind und bevorzugt als Lagesensoren, optische Sensoren und/oder bildgebende Sensoren (46) ausgebildet sind.

3. Vorrichtung gemäß Anspruch 1 oder 2, wobei auf eine Patientenliege (3) und/oder einen Patientenstuhl wirkende Aktoren vorgesehen sind, über welche der Patient (2) seine Lage selbst einstellen kann.

4. Vorrichtung gemäß einem der vorstehenden Ansprüche, wobei eine Datenverarbeitungsvorrichtung (5) zur Auswertung der Signale der Lageermittelungsmittel (40, 42, 44, 46) vorgesehen ist, wobei die Datenverarbeitungsvorrichtung (5) dazu ausgebildet ist, die Entwässerungsrate, bevorzugt die Ultrafiltrationsrate (UFR), entsprechend der ermittelten Lage des Patienten (2) einzustellen, zu regeln und/oder zu steuern.

5. Vorrichtung gemäß einem der vorstehenden Ansprüche, wobei die Blutbehandlungsvorrichtung (1) zur Durchführung der Hämodialyse, der Hämofiltration, der Hämodiafiltration, der Peritonealdialyse, der Apherese und/oder der Plasmapherese ausgebildet ist.

6. Vorrichtung gemäß einem der vorstehenden Ansprüche, wobei die Vorrichtung dazu eingerichtet ist, die Ermittlung der Lage des Patienten (2) an einer Patientenliege (3), an einem Patientenstuhl und/oder an dem Patienten (2) selbst durchzuführen, bevorzugt über die Auswertung von an der Patientenliege (3) und/oder dem Patientenstuhl angebrachten Sensoren (40, 42, 44), von am Patienten (2) angeordneten Sensoren und/oder von den Patienten (2) überwachenden Sensoren, insbesondere der Auswertung von Lagesensoren (40, 42, 44), optischen Sensoren und/oder bildgebenden Sensoren (46).

7. Vorrichtung gemäß einem der vorstehenden Ansprüche, wobei die Blutbehandlungsvorrichtung (1) zur Durchführung einer Peritonealdialyse ausgebildet ist, und die Vorrichtung so eingerichtet ist, dass die Entwässerungsrate durch Einstellen, Steuern und/oder Regeln der Konzentration gelöster Stoffe in der Dialyseflüssigkeit, bevorzugt von Glukose und/oder Dextrin, vor dem Einbringen der Dialyseflüssigkeit in die Bauchhöhle eingestellt, gesteuert und/oder geregelt wird.

8. Vorrichtung gemäß Anspruch 7 wobei die Vorrichtung so eingerichtet ist, dass bei einer Änderung der Lage des Patienten (2) die in der Bauchhöhle vorhandene Dialyseflüssigkeit gegen eine Dialyseflüssigkeit ausgetauscht wird, welche eine andere Konzentration gelöster Stoffe aufweist.

9. Vorrichtung gemäß einem der vorstehenden Ansprüche, wobei die Blutbehandlungsvorrichtung (1) zur Durchführung einer Ultrafiltration ausgebildet ist und die Vorrichtung so eingerichtet ist, dass die Entwässerungsrate durch die Ultrafiltrationsrate (UFR) einstellbar ist und die Ultrafiltrationsrate (UFR) abhängig von der ermittelten Lage des Patienten (2) eingestellt, gesteuert und/oder geregelt wird.

10. Vorrichtung gemäß Anspruch 9 wobei die Vorrichtung so eingerichtet ist, dass die Einstellung, Steuerung und/oder Regelung der Ultrafiltrationsrate (UFR) durch Einstellung, Steuerung und/oder Regelung der Pumprate bei der Ultrafiltration erreicht wird, insbesondere durch Einstellung, Steuerung und/oder Regelung der Pumprate einer stromabwärts eines Dialysefilters angeordneten Schlauchrollenpumpe, Zahnradpumpe, Membranpumpe und/oder Impellerpumpe.

11. Vorrichtung gemäß einem der vorstehenden Ansprüche, wobei die Vorrichtung so eingerichtet ist, dass die Entwässerungsrate, bevorzugt die Ultrafiltrationsrate, bei einem im Wesentlichen liegenden Patienten (2) höher eingestellt, gesteuert und/oder geregelt wird, als bei einem gegenüber der liegenden Position aufrechter angeordneten Patienten (2) und/oder wobei die Vorrichtung so eingerichtet ist, dass die Entwässerungsrate, bevorzugt die Ultrafiltrationsrate, zu Beginn der Behandlung höher eingestellt, gesteuert und/oder geregelt wird, als in einem nachfolgenden Abschnitt der Behandlung, und/oder wobei die Vorrichtung so eingerichtet ist, dass die Entwässerungsrate, bevorzugt die Ultrafiltrationsrate, nur in einem ersten Behandlungsabschnitt abhängig von der Lage des Patienten (2) gesteuert und/oder geregelt wird, in einem zweiten Behandlungsabschnitt jedoch konstant gehalten wird, wobei der erste Behandlungsabschnitt bevorzugt durch das Entziehen der Hälfte der geplanten Flüssigkeitsmenge (UFG/2) endet.

12. Vorrichtung gemäß einem der vorstehenden Ansprüche, wobei die Vorrichtung so eingerichtet ist, dass die Entwässerungsrate, bevorzugt die Ultrafiltrationsrate, zur Einstellung, Steuerung und/oder Regelung abhängig von der Lage des Patienten (2) um eine vorgegebene Differenz (UFR+) von einer vorgegebenen Entwässerungsrate, bevorzugt Ultrafiltrationsrate (UFR_{SET}), angehoben und/oder abgesenkt wird, wobei die vorgegebene Differenz (UFR+) bevorzugt aus der zu erwartenden Plasmavolumenänderung zwischen im Wesentlichen liegender und im Wesentlichen aufrechter Position des Patienten (2) abgeleitet wird.

13. Vorrichtung gemäß einem der vorstehenden Ansprüche, wobei die Vorrichtung so eingerichtet ist, dass die Entwässerungsrate, bevorzugt die Ultrafiltrationsrate (UFR), zunächst für einen Zeitabschnitt (t_{UFR±}) der Behandlung angehoben wird, bevorzugt um einen vorgegebenen Wert (UFR+), und dann bei Auftreten einer Lageänderung des Patienten in Richtung einer aufrechteren Lage, für den gleichen Zeitabschnitt (t_{UFR±}) abgesenkt wird, bevorzugt um den gleichen vorgegebenen Wert (UFR+), wobei bevorzugt zunächst eine erhöhte Entwässerungsrate, bevorzugt Ultrafiltrationsrate (UFR=UFR_{SET} + UFR+), und dann eine erniedrigte Entwässerungsrate, bevorzugt Ultrafiltrationsrate (UFR=UFR_{SET} - UFR+), eingestellt, geregelt und/oder gesteuert wird.

14. System zur Blutbehandlung umfassend eine Blutbehandlungsvorrichtung (1) zum Entwässern des Blutes eines Patienten (2) bei einer einstellbaren Entwässerungsrate, sowie eine Patientenliege (3) und/oder einen Patientenstuhl zur Lagerung des Patienten (2) während der Blutbehandlung,
**gekennzeichnet durch**
eine Vorrichtung gemäß einem der vorstehenden Ansprüche.

## Claims

1. Device for blood treatment of a patient (2), comprising a blood treatment device (1) for dewatering of the blood of the patient (2) at an adjustable dewatering rate, **characterised by** position determination means (40, 42, 44, 46) for determining the position of the patient (2), wherein the dewatering rate is adjustable, regulatable and/or controllable depending on the determined position of the patient.

2. Device according to claim 1, wherein the position determination means are formed as sensors (40, 42, 44) attached to a patient bed (3) and/or patient chair, as sensors arranged on the patient (2) and/or as sensors monitoring the patient and are preferably formed as position sensors, optical sensors and/or imaging sensors (46).

3. Device according to claim 1 or 2, wherein actuators acting on a patient bed (3) and/or a patient chair are provided, via which the patient (2) can adjust his position himself.

4. Device according to any one of the preceding claims, wherein a data processing device (5) for analysis of the signals of the position determination means (40, 42, 44, 46) is provided, wherein the data processing device (5) is adapted to adjust, to regulate and/or to control the dewatering rate, preferably the ultrafiltration rate (UFR), corresponding to the determined position of the patient (2).

5. Device according to any one of the preceding claims, wherein the blood treatment device (1) is adapted to implement haemodialysis, haemofiltration, haemodiafiltration, peritoneal dialysis, apheresis and/or plasmapheresis.

6. Device according to any one of the preceding claims, wherein the device is configured to implement the determination of the position of the patient (2) on a patient bed (3), on a patient chair and/or on the patient (2) himself, preferably via the analysis of sensors (40, 42, 44) attached to the patient bed (3) and/or to the patient chair, of sensors arranged on the patient (2) and/or of sensors monitoring the patient (2), in particular the analysis of position sensors (40, 42, 44), optical sensors and/or imaging sensors (46).

7. Device according to any one of the preceding claims, wherein the blood treatment device (1) is adapted to implement peritoneal dialysis, and the device is configured such that the dewatering rate is adjusted, controlled and/or regulated by adjusting, controlling and/or regulating the concentration of dissolved substance in the dialysis fluid, preferably of glucose and/or dextrin, before the introduction of the dialysis fluid into the abdominal cavity.

8. Device according to claim 7, wherein the device is configured such that, in the case of a change of the position of the patient (2), the dialysis fluid present in the abdominal cavity is exchanged for a dialysis fluid which has a different concentration of dissolved substance.

9. Device according to any one of the preceding claims, wherein the blood treatment device (1) is adapted to implement an ultrafiltration and the device is configured such that the dewatering rate is adjustable using the ultrafiltration rate (UFR) and the ultrafiltration rate (UFR) is adjusted, controlled and/or regulated depending on the determined position of the patient (2).

10. Device according to claim 9, wherein the device is configured such that the adjustment, control and/or regulation of the ultrafiltration rate (UFR) is achieved by adjusting, controlling and/or regulating the pump rate during the ultrafiltration, in particular by adjusting, controlling and/or regulating the pump rate of a peristaltic pump, gear pump, diaphragm pump and/or impeller pump arranged downstream of a dialysis filter.

11. Device according to any one of the preceding claims, wherein the device is configured such that the dewatering rate, preferably the ultrafiltration rate, is adjusted, controlled and/or regulated to be higher in the case of a substantially lying patient (2) than in the case of a patient (2) arranged to be more upright compared to the lying position, and/or wherein the device is configured such that the dewatering rate, preferably the ultrafiltration rate, is adjusted, controlled and/or regulated to be higher at the beginning of the treatment than in a subsequent period of the treatment, and/or wherein the device is configured such that the dewatering rate, preferably the ultrafiltration rate, is controlled and/or regulated only in a first treatment period depending on the position of the patient (2), but is kept constant in a second treatment period, wherein the first treatment period preferably ends by the removal of half of the planned fluid quantity (UFG/2).

12. Device according to any one of the preceding claims, wherein the device is configured such that the dewatering rate, preferably the ultrafiltration rate, is increased and/or decreased by a predetermined difference (UFR+) of a predetermined dewatering rate, preferably ultrafiltration rate (UFR_{SET}), depending on the position of the patient (2) to adjust, control and/or regulate, wherein the predetermined difference (UFR+) is preferably derived from the expected plasma volume change between a substantially lying and a substantially upright position of the patient (2).

13. Device according to any one of the preceding claims, wherein the device is configured such that the dewatering rate, preferably the ultrafiltration rate (UFR), is firstly increased for a period of time (t_{UFR±}) of the treatment, preferably by a predetermined value (UFR+), and then, in the case of occurrence of a position change of the patient in the direction of an upright position, is decreased for the same period of time (t_{UFR±}), preferably by the same predetermined value (UFR+), wherein preferably firstly an increased dewatering rate, preferably ultrafiltration rate (UFR = UFR_{SET} + UFR+), and then a decreased dewatering rate, preferably ultrafiltration rate (UFR = UFR_{SET} - UFR+), is adjusted, regulated and/or controlled.

14. System for blood treatment comprising a blood treatment device (1) for dewatering the blood of a patient (2) in the case of an adjustable dewatering rate, as well as a patient bed (3) and/or a patient chair for positioning of the patient (2) during the blood treatment, **characterised by** a device according to any one of the preceding claims.

## Revendications

1. Dispositif servant au traitement du sang d'un patient (2), comprenant un dispositif de traitement du sang (1) servant à retirer l'eau du sang du patient (2) à un taux de retrait d'eau pouvant être réglé,
**caractérisé par**
des moyens de détermination de position (40, 42, 44, 46) servant à déterminer la position du patient (2), dans lequel le taux de retrait d'eau peut être réglé, régulé et/ou commandé en fonction de la position déterminée du patient.

2. Dispositif selon la revendication 1, dans lequel les moyens de détermination de position sont réalisés sous la forme de capteurs (40, 42, 44) installés au niveau d'un lit pour patient (3) et/ou d'un fauteuil pour patient, sous la forme de capteurs disposés au niveau du patient (2) et/ou sous la forme de capteurs surveillant le patient et sont réalisés de manière préférée sous la forme de capteurs de position, de capteurs optiques et/ou de capteurs d'imagerie (46).

3. Dispositif selon la revendication 1 ou 2, dans lequel sont prévus des actionneurs agissant sur un lit pour patient (3) et/ou sur un fauteuil pour patient, par l'intermédiaire desquels le patient (2) peut régler lui-même sa position.

4. Dispositif selon l'une quelconque des revendications précédentes, dans lequel un dispositif de traitement de données (5) est prévu pour évaluer les signaux des moyens de détermination de position (40, 42, 44, 46), dans lequel le dispositif de traitement de données (5) est réalisé pour régler, réguler et/ou commander le taux de retrait d'eau, de manière préférée le taux d'ultrafiltration (UFR) selon la position déterminée du patient (2).

5. Dispositif selon l'une quelconque des revendications précédentes, dans lequel le dispositif de traitement du sang (1) est réalisé pour effectuer l'hémodialyse, l'hémofiltration, l'hémodiafiltration, la dialyse péritonéale, l'aphérèse et/ou la plasmaphérèse.

6. Dispositif selon l'une quelconque des revendications précédentes, dans lequel le dispositif est mis au point pour effectuer la détermination de la position du patient (2) au niveau d'un lit pour patient (3), au niveau d'un fauteuil pour patient et/ou au niveau du patient (2) lui-même, de manière préférée par l'intermédiaire de l'évaluation de capteurs (40, 42, 44) installés au niveau du lit pour patient (3) et/ou du fauteuil pour patient, de capteurs disposés au niveau du patient (2) et/ou de capteurs surveillant le patient (2), en particulier l'évaluation de capteurs de position (40, 42, 44), de capteurs optiques et/ou de capteurs d'imagerie (46).

7. Dispositif selon l'une quelconque des revendications précédentes, dans lequel le dispositif de traitement du sang (1) est réalisé pour effectuer une dialyse péritonéale, et le dispositif est mis au point de sorte que le taux de retrait d'eau est réglé, commandé et/ou régulé en réglant, commandant et/ou régulant la concentration de substances dissoutes dans le liquide de dialyse, de manière préférée de glucose et/ou de dextrine, avant l'introduction du liquide de dialyse dans la cavité abdominale.

8. Dispositif selon la revendication 7, dans lequel le dispositif est mis au point de sorte que dans le cas d'un changement de la position du patient (2), le liquide de dialyse présent dans la cavité abdominale est remplacé par un liquide de dialyse, qui présente une concentration autre de substances dissoutes.

9. Dispositif selon l'une quelconque des revendications précédentes, dans lequel le dispositif de traitement du sang (1) est réalisé pour effectuer une ultrafiltration, et le dispositif est mis au point de sorte que le taux de retrait d'eau peut être réglé par le taux d'ultrafiltration (UFR) et le taux d'ultrafiltration (UFR) est réglé, commandé et/ou régulé en fonction de la position déterminée du patient (2).

10. Dispositif selon la revendication 9, dans lequel le dispositif est mis au point de sorte que le réglage, la commande et/ou la régulation du taux d'ultrafiltration (UFR) sont atteints par le réglage, la commande et/ou la régulation du taux de pompage lors de l'ultrafiltration, en particulier par le réglage, la commande et/ou la régulation du taux de pompage d'une pompe péristaltique, d'une pompe à engrenages, d'une pompe à membrane et/ou d'une pompe à rotor disposée en aval d'un filtre de dialyse.

11. Dispositif selon l'une quelconque des revendications précédentes, dans lequel le dispositif est mis au point de sorte que le taux de retrait d'eau, de manière préférée le taux d'ultrafiltration, est réglé, commandé et/ou régulé à un niveau plus élevé lorsque le patient (2) est sensiblement couché que lorsque le patient (2) se trouve en position debout par rapport à la position couchée, et/ou dans lequel le dispositif est mis au point de sorte que le taux de retrait d'eau, de manière préférée le taux d'ultrafiltration, est réglé, commandé et/ou régulé à un niveau plus élevé au début du traitement que dans une phase qui suit du traitement, et/ou dans lequel le dispositif est mis au point de sorte que le taux de retrait d'eau, de manière préférée le taux d'ultrafiltration, est commandé et/ou régulé seulement lors d'une première phase du traitement en fonction de la position du patient (2), est maintenu toutefois constant lors d'une deuxième phase du traitement, dans lequel la première phase de traitement se termine de manière préférée par l'élimination de la moitié de la quantité de liquide (UFG/2) prévue.

12. Dispositif selon l'une quelconque des revendications précédentes, dans lequel le dispositif est mis au point de sorte que le taux de retrait d'eau, de manière préférée le taux d'ultrafiltration, est, aux fins du réglage, de la commande et/ou de la régulation en fonction de la position du patient (2), augmenté et/ou baissé d'une différence (UFR+) spécifiée d'un taux de retrait d'eau spécifié, de manière préférée d'un taux d'ultrafiltration (UFR_{SET}), dans lequel la différence (UFR+) spécifiée est déduite de manière préférée du changement de volume de plasma attendu entre la position sensiblement couchée et la position sensiblement debout du patient (2).

13. Dispositif selon l'une quelconque des revendications précédentes, dans lequel le dispositif est configuré de sorte que le taux de retrait d'eau, de manière préférée le taux d'ultrafiltration (UFR), est augmenté en premier lieu pour un laps de temps (t_{UFR±}) du traitement, de manière préférée d'une valeur (UFR+) spécifiée, puis est baissé pour le même laps de temps (t_{UFR±}) lors de la survenue d'un changement de position du patient en direction d'une position debout, de manière préférée de la même valeur (UFR+) spécifiée, dans lequel de manière préférée un taux de retrait d'eau élevé, de manière préférée un taux d'ultrafiltration (UFR = UFR_{SET} + UFR+) est réglé, régulé et/ou commandé en premier lieu, puis un taux de retrait d'eau plus bas, de manière préférée un taux d'ultrafiltration (UFR = UFR_{SET} - UFR+) est réglé, régulé et/ou commandé.

14. Système servant au traitement du sang comprenant un dispositif de traitement du sang (1) servant à retirer l'eau du sang d'un patient (2) à un taux de retrait d'eau pouvant être réglé, ainsi qu'un lit pour patient (3) et/ou un fauteuil pour patient servant à positionner le patient (2) au cours du traitement du sang,
**caractérisé par**
un dispositif selon l'une quelconque des revendications précédentes.
